# EUROPEAN PATENT SPECIFICATION

(11) **EP 0 759 746 B1**
(45) Date of publication and mention of the grant of the patent: **27.12.2000**
(21) Application number: 95918693.3
(22) Date of filing: 17.05.1995
(51) Int. Cl.: A61K 9/70

(54) **A MEDICATED WIPE**
MEDIZINISCHES IMPRÄGNIERTES WISCHTUCH
TAMPON DE TISSU IMPREGNE PAR UN AGENT PHARMACEUTIQUE

(30) Priority: 17.05.1994 GB 9409945
(43) Date of publication of application: 05.03.1997
(73) Proprietor: BIOGLAN LABORATORIES LTD., Hitchin Hertfordshire SG4 0TJ (GB)
(72) Inventor: GOODMAN, Michael, Ampthill Bedfordshire MK45 2XE (GB)
(74) Representative: Jump, Timothy John Simon
(86) International application number: GB9501108
(87) International publication number: WO9531189

(56) References cited:
- EP-A- 0 001 733
- EP-A- 0 586 282
- WO-A-91/04730
- DE-A- 4 315 866
- BRITISH JOURNAL OF DERMATOLOGY, vol. 102, no. 6, 1980 pages 659-663, D. MURRAY ET AL 'A CONTROLLED TRIAL OF PHOTOCHEMOTHERAPY FOR PERSISTENT PALMOPLANTAR PUSTULOSIS'
- EXCERPTA MEDICA (INT. CONGR. SER.), vol. 1011, 1992 pages 521-525, O. BECH ET AL 'PHOTOSENSITISATION OF TUMOR CELLS INVITRO AND IN VIVO USING ENDOGENOUS PORPHYRINS INDUCED WITH EXOGENOUS 5-AMINOLEVULINIC ACID'
- PATENT ABSTRACTS OF JAPAN vol. 007 no. 131 (C-0169) ,8 June 1983 & JP,A,58 046017 (NITTO DENKI KOGYO KK) 17 March 1983, & DATABASE WPI Week 8317 Derwent Publications Ltd., London, GB; AN 83-40335 & JP-A-58 046 017 (NITTO ELECTRIC IND KK) , 17 March 1987
- J.E.F. REYNOLDS, ED. 'MARTINDALE, THE EXTRA PHARMACOPOEIA' 1993 , THE PHARMACEUTICAL PRESS , LONDON see page 763 - page 765
- DATABASE WPI Week 9314 Derwent Publications Ltd., London, GB; AN 93-111902 & JP-A-05 049 551 (JEX KK) , 2 March 1993 & PATENT ABSTRACTS OF JAPAN vol. 017 no. 349 (C-1078) ,2 July 1993 & JP,A,05 049551 (JIEKUSU KK) 2 March 1993,
- PATENT ABSTRACTS OF JAPAN vol. 008 no. 145 (C-232) ,6 July 1984 & JP,A,59 053409 (NITTO DENKI KOGYO KK) 28 March 1984, & DATABASE WPI Week 8419 Derwent Publications Ltd., London, GB; AN 84-116608 & JP-A-59 053 409 (NITTO ELECTRIC IND KK) , 28 March 1984

## Description

This invention relates to an absorbent wipe or swab, impregnated with a pharmaceutically active agent, or other material, for use in applying, by wiping, the agent or other material to the skin.

Medical or cleansing wipes, each comprising an absorbent woven or non-woven fabric or cloth, a woven or non-woven cellulosic tissue or web, or the like, impregnated with an aqueous or alcoholic solution of a detergent, perfume, antiseptic, a locally active anaesthetic, an antihistamine, a rubefacient, or a carboxylic acid (as a virucide), are known and disclosed in at least British Patent Nos. 1565775; 2103089 and; 2190289. Such wipes are normally supplied individually wrapped in sealed sachets or pockets which, for example, can be formed by sandwiching an impregnated and folded wipe between two larger sheets of an aluminium foil/polyethylene film laminate and heat welding the sheets of laminate together, around the periphery of the folded wipe.

Medicated and non-medicated emollient compositions, for use in treating dermatological disorders in which the abnormal skin is exposed to ultraviolet light (UV), preferably to UVA, are disclosed in International Patent Application No. PCT/GB92/00556. Such compositions can include, as active agents, substances effective in stimulating melanocytes to produce melanin, such as a psoralen or urocanic acid, the preferred psoralens being 8-methoxypsoralen and trimethylpsoralen. The described emollient compositions are lipophilic non-viscous liquids which, on application to skin or a like surface, spread to provide a substantially uniform coating of lipophilic emollient, which is sufficiently non-volatile to persist, once spread, for a significant period of time. The compositions can comprise a lipophilic emollient in admixture with a polar solvent and a surfactant.

When used, medicated emollients of the aforementioned type are generally spread over an extensive area of the body and it has been proposed, therefore, to apply the same using spray applicators (see the aforementioned International Patent Application). However, if the emollient composition includes an active agent, such as 8-methoxypsoralen, which has the potential for being systemically toxic, it has now been found that this method of application can be significantly disadvantaged.

For example, precautions must be taken to prevent the aerosol, produced by the applicator, from entering the eyes and other body orifices of patients and healthcare workers exposed thereto. Also, in order to prevent prolonged and uncontrollably repeated exposure to psoralens, which can lead to the absorption of harmful quantities of such an agent, healthcare workers who regularly treat patients with compositions including psoralens, using spray applicators, must wear cumbersome protective clothing. The risks, in fact, are of sufficient significance to make it unsafe for patients to use spray applicators for self-applying such medicated compositions.

The present invention is intended to offer a solution to at least some of these problems.

JP-A-58046017 discloses the incorporation of an active agent into the pressure-sensitive layer of a sticking plaster. The plaster is intended for application to diseased skin for the latter to be irradiated through the plaster with ultraviolet light. It is suggested that the described plaster offers a simple treatment and has the advantage that it can be peeled off cleanly without leaving any residue on the skin, after treatment.

The possibility of painting the skin with an emulsion comprising 8-methoxypsoralen, using cotton wool on a stick, is disclosed in British Journal of Dermatology, Vol. 102, No. 6, 1980, pages 659-663. This article provides no details of the emulsion employed and suggests that there are significant disadvantages associated with the topical application of 8-methoxypsoralen, in comparison to oral administration thereof.

EP-A-0001733 discloses compositions comprising 5-methoxy psoralen and suggests that this agent is more active than other psoralens and less prone to elicit unwanted side effects. Similar compositions are disclosed in EP-A-0586282.

DE-A-4315866 discloses a viscous cream composition which can include a psoralen, as an active agent.

JP-A-5049551 discloses a paper substrate impregnated with a liquid paraffin based composition which includes gamma-linolenic acid, as an active agent, that is specifically for cleaning the area surrounding the anus.

JP-A-59053409 discloses compositions comprising a siloxane, an amide, such as DMF, and an active agent and includes a suggestion that the described compositions may be absorbed in a gauze.

According to the present invention there is provided a wipe comprising an absorbant woven or non-woven fabric, cloth or tissue substrate, impregnated with an emollient composition comprising a lipophilic emollient and a pharmaceutically active agent, wherein the active agent is a substance effective in stimulating melanocytes to produce melanin and/or is effective in a topical treatment of a skin condition in combination with therapeutic eletromagnetic radiation falling in the range of 220-700nm, and is selected from 8-methoxypsoralen, trimethylpsoralen, 6-methoxypsoralen, 3-carbethoxypsoralen, cis-urocanic acid, trans-urocanic acid, mixtures of cis- and trans-urocanic acids and amino oxoaliphatic carboxylic acids in which the aliphatic skeleton has 4-10 carbon atoms, the amino group is primary, or is a secondary or tertiary alkyl amino group, and is γ, or preferably δ, or further from the carboxy carbon and the oxo group is β, or preferably γ, or further from the carboxy carbon, the composition is a non-viscous liquid which, on application to skin, spreads to provide a substantially uniform coating of the lipophilic emollient and active agent, and the coating does not absorb a significant amount of incident therapeutic eletromagnetic radiation within the range of 220-700nm and is sufficiently non-volatile to persist for a period of sufficient length, for a therapeutically effective dose of said electromagnetic radiation to be administered.

Wipes in accordance with the invention can be employed to apply the pharmaceutically active agent, or emollient composition, to the skin, with minimal risk of the applied material accidentally entering the eyes or other body orifices of either patients or healthcare workers. Thus, even when an active agent having the potential to be systemically toxic is so applied, the need for protective clothing is minimised; the maximum protection required to use a wipe in accordance with the invention can be as little as a surgical or like glove, worn on the hand in which the wipe is held.

In an embodiment of the invention, the pharmaceutically active agent is selected from 8-methoxypsoralen, trimethylpsoralen, 6-methoxypsoralen, 3-carbethoxypsoralen, cis-urocanic acid, trans-urocanic acid, mixtures of cis- and trans-urocanic acids, and amino oxoaliphatic carboxylic acids wherein the aliphatic skeleton has 4-10 carbon atoms (including the carboxy carbon), the amino group is primary, or is a secondary or a tertiary alkyl amino group, and is γ, or preferably δ, or further from the carboxy carbon and the oxo group is β, or preferably γ, or further from the carboxy carbon. The preferred δ-amino oxoaliphatic acid is δ-aminolevulinic acid (5-amino, 4-oxopentanoic acid). The active agent, preferably, is dissolved in a suitable carrier or solvent. Preferably, the aliphatic skeleton has 5-10 carbon atoms, more preferably 5-8 carbon atoms and most preferably 5 or 6 carbon atoms.

Preferably, wipes in accordance with the invention are suitable for use in the topical treatment of psoriasis, or other skin disorders in which the stratum corneum becomes flaky or scaly, including mycoses fungicides, and acne vulgaris, alopeica areata, dermatitis herpetiformis, eosinophilic pustular folliculitis, erythrokeratoderma (symmetrical and progressive), chronic lichenoid GVH disease, granuloma annulare, histiocytosis X, ichthyosis linearis circumflexa, lichen planus, pityriasis lichenoides, pityriasis rosea, pityriasis rubra pilaris, pressure sores, pruritis (primary and secondary), seleromyxoedema, subcorneal pustular dermatoses, transient acantholytic dermatoses, and atopic eczema.

The method of treatment employed can be as described in International Patent Application No. GB92/00556.

Where the active agent is a δ-amino oxoaliphatic carboxylic acid, such as δ-aminolevulinic acid, the electromagnetic radiation used, preferably, is in the visible spectrum, more preferably excludes any UV radiation and, most preferably, is largely in the range of 600-700nm. Such active agents can be employed in emollient compositions which absorb significant amounts of UV radiation.

In a preferred embodiment of the invention, the pharmaceutically active agent is dispersed or dissolved in an emollient composition a type disclosed in International Application No. PCT/GB92/00556.

Thus, the emollient composition can comprise a non-volatile and relatively thick lipophilic emollient dissolved in a carrier. Preferably, after application to healthy normal skin, the emollient transmits through to the skin 90% or more and, more preferably, 95% or more incident UVA, UVB or broad band UV.

Preferably, the lipophilic emollient is chosen so that a 5µm layer thereof absorbs 20% or less and, preferably, 10% or less of the incident therapeutic radiation. Preferably, a 5µm layer of the lipophilic emollient absorbs 20% or less and, more preferably, 10% or less incident radiation at any wavelength within the broad band UV, UVA or UVB regions of the electromagnetic spectrum.

In this specification broad band UV is defined as radiation of a wavelength between 285 and 400 nm, UVA has a wavelength of 320-400 nm and UVB a wavelength of 285-320 nm.

In a preferred embodiment the composition has a viscosity of between 5 and 20,000 centipoise and, preferably, a viscosity of between 5 and 2,500 centipoise. Preferably, the lipophilic emollient has a partial vapour pressure of 17.5mmHg, or less, and preferably, 10mmHg, or less, at 20°C. Also, it is preferred that the partial vapour pressure of a coating formed by the composition on skin or a like surface, should be 17.5mmHg, or less, and preferably, 10mmHg, or less, at 20°C, for a period of sufficient length for a therapeutically effective dose of incident radiation to be administered.

The lipophilic emollient is, preferably, in admixture with a carrier having the property of enhancing the spreadability of the emollient composition, to assist in the formation of an even and unbroken coating of the lipophilic emollient. The preferred carriers are non-polar solvents, including volatile silicone oils, such as cyclomethicone, octamethylcyclotetrasiloxane (ABIL K4, available from Goldschmidt GmbH of Essen, Germany), decamethylcyclopentasiloxane (ABIL B8839, available from Goldschmidt GmbH of Essen, Germany), a dimethicone, or a mixture of any of these. The carrier can comprise a surfactant, which can be isopropylisostearate, pentaerythratol tetraisostearate, promyristyl propionate, myristyl lactate, oleyl erucate, isopropyl myristate, isocetyl stearate, isopropyl isostearate, other aliphatic esters of fatty acids or a mixture of any of these.

The lipophilic emollient, preferably, is coconut oil, sesame oil, sunflower oil, corn oil, a mineral oil, such as liquid paraffin or a fraction thereof, or any other like saturated oil or a mixture of any of these, and, more preferably, is coconut oil.

Most preferably, the pharmaceutically active agent is carried in a vehicle which includes singly or a mixture of:-
(i) a vegetable oil or oils, such as coconut arachis or palm kernel;
(ii) a mineral oil or oils;
(iii) a volatile silicone oil or oils, such as octamethylcyclotetrasiloxane or decamethylcyclopentasiloxane; and
(iv) an aliphatic ester of a fatty acid, such as isopropyl myristate or isopropyl isostearate.

In further embodiments, a coloured or UV disclosing dye is included with the pharmaceutically active agent, to act as a marker, showing where the latter has been applied.

The preferred material for forming the wipe substrate is cotton lint and the impregnated wipe is, preferably, sealed into an enveloping sachet or pocket. Preferably, the sachet or pocket is formed by sealingly sandwiching a folded and impregnated wipe between two sheets of an aluminium foil/polyethylene film laminate. The sheets of laminate may comprise folded over portions of a single sheet of such material.

The preferred psoralen is 8-methoxypsoralen or trimethylpsoralen and is included in a concentration of between 0.0125% and 10% and, preferably, 0.0125% and 0.1% by weight. The other listed active agents can be used in similar amounts, although the δ-amino oxoaliphatic carboxylic acids can be used in concentrations of up to 15% by weight.

Specific embodiments of the present invention will now be described, by way of illustration only.

### Example 1

100gms of natural coconut oil is blended with 100gms of isopropyl isostearate and 100gms of cyclomethicone, to provide a non-viscous liquid composition. The composition is divided into four equal parts and 8-methoxypsoralen was added to these in the following amounts:-
(a) 0.0125% by weight;
(b) 0.025% by weight;
(c) 0.05% by weight and;
(d) 0.1% by weight.

4ml aliquots of each of the resulting solutions (a)-(d) are deposited onto 10cm x 10cm cotton lint sheets and each sheet is then sealed into a pouch, formed from a laminate of aluminium foil and polyethylene film.

### Example 2

A non-viscous liquid composition, prepared in the manner described in Example 1, is divided into four equal parts and δ-aminolevulinic acid was added to these in the following amounts:
(a) 0.05% by weight
(b) 1% by weight
(c) 5% by weight
(d) 10% by weight.

4ml aliquots of each of the resulting solutions (a)-(d) are deposited onto 10cm x 10cm cotton lint sheets and each sheet is sealed into a pouch, formed from a laminate of aluminium foil and polyethylene film.

### Example 3

To use a wipe, prepared in the manner described in Example 1 or 2, it should be removed from its pouch or sachet and wiped over the area to be treated with incident electromagnetic radiation, in order to apply a thin and uniform coating of the coconut oil and 8-methoxypsoralen, or δ-aminolevulinic acid.

Once coated in a composition, prepared in accordance with the instructions set out in Example 1, a patient, suffering from psoriasis, is subjected to UVA treatment of between 2 and 5 minutes in a conventional UVA light cabinet, after which the composition may be washed off. Treatment normally involves 2-3 such exposures for 4-8 weeks, until the diseased areas have reverted substantially back to normal. Thereafter, the disease may be held in check by a maintenance regime of 1 PUVA treatment per week over an indefinite period.

Whole body light cabinets which are suitable for use in the manner set out are listed in P.J. Mounford, "Phototherapy and PhotoChemotherapy Ultraviolet Irradiation Equipment", Photodermatology 1986:3:83/91. The exact exposure times for each particular light cabinet should be determined by a responsible clinician in the normal manner.

Psoriasis patients can be treated in a similar manner with compositions prepared in accordance with the instructions set out in Example 2, excepting that the radiation employed is in the visible spectral region and can be applied at a dose of between 10 and 50 J/m² at a power density of about 70mW/cm².

## Claims

1. A wipe comprising an absorbant woven or non-woven fabric, cloth or tissue substrate, impregnated with an emollient composition comprising a lipophilic emollient and a pharmaceutically active agent, wherein the active agent is a substance effective in stimulating melanocytes to produce melanin and/or is effective in a topical treatment of a skin condition in combination with therapeutic eletromagnetic radiation falling in the range of 220-700nm, and is selected from 8-methoxypsoralen, trimethylpsoralen, 6-methoxypsoralen, 3-carbethoxypsoralen, cis-urocanic acid, trans-urocanic acid, mixtures of cis- and trans-urocanic acids and amino oxoaliphatic carboxylic acids in which the aliphatic skeleton has 4-10 carbon atoms, the amino group is primary, or is a secondary or tertiary alkyl amino group, and is γ, or preferably δ, or further from the carboxy carbon and the oxo group is β, or preferably γ, or further from the carboxy carbon, the composition is a non-viscous liquid which, on application to skin, spreads to provide a substantially uniform coating of the lipophilic emollient and active agent, and the coating does not absorb a significant amount of incident therapeutic eletromagnetic radiation within the range of 220-700nm and is sufficiently non-volatile to persist for a period of sufficient length, for a therapeutically effective dose of said electromagnetic radiation to be administered.

2. A wipe as claimed in claim 1, wherein the amino oxoaliphatic acid is a δ-amino oxoaliphatic acid.

3. A wipe as claimed in claim 2, wherein the amino oxoaliphatic acid is δ-aminolevulinic acid.

4. A wipe as claimed in claim 2 or claim 3, wherein the therapeutic electromagnetic radiation is in the visible spectrum.

5. A wipe as claimed in claim 4, wherein the therapeutic radiation is in the range of 600-700mn.

6. A wipe as claimed in claim 1, wherein the pharmaceutically active agent is 8-methoxypsoralen.

7. A wipe as claimed in claim 6, wherein the therapeutic electromagnetic radiation is UV, in the range 285-400nm.

8. A wipe as claimed in claim 7, wherein the therapeutic radiation is UVA, in the range 320-400nm.

9. A wipe as claimed in claim 1, wherein the therapeutic eletromagnetic radiation is broad band UV, UVA or UVB radiation, the emollient composition is anhydrous and has a viscosity of between 5 and 2500 centipoise, and the lipophilic emollient, when spread in a 5µm layer, absorbs 10% or less of the incident therapeutic eletromagnetic radiation and has a partial vapour pressure of 10mmHg or less, at 20°C.

10. A wipe as claimed in any of claims 1-8, wherein a 5µm layer of the lipophilic emollient absorbs 20% or less incident therapeutic eletromagnetic radiation.

11. A wipe as claimed in claim 10, wherein a 5µm layer of the lipophilic emollient absorbs 10% or less incident therapeutic eletromagnetic radiation.

12. A wipe as claimed in any of claims 1-8, 10 and 11, wherein the emollient composition has a viscosity of between 5 and 20,000 centipoise.

13. A wipe as claimed in claim 12, wherein the emollient composition has a viscosity of between 5 and 2500 centipoise.

14. A wipe as claimed in any of claims 1-8 and 10-13, wherein the lipophilic emollient has a partial vapour pressure of 17.5mmHg or less at 20°C.

15. A wipe as claimed in claim 14, wherein the lipophilic emollient has a partial vapour pressure of 10 mmHg or less at 20°C.

16. A wipe as claimed in any of claims 1-8 and 10-15, wherein the partial vapour pressure of a coating, formed by the emollient composition on skin, is 17.5mmHg or less at 20°C.

17. A wipe as claimed in claim 16, wherein the partial vapour pressure of a coating, formed by the emollient composition on the skin is 10 mmHg or less at 20°C.

18. A wipe as claimed in any of claims 1-17, wherein the emollient composition further comprises a carrier for the lipophilic emollient.

19. A wipe as claimed in claim 18, wherein the carrier comprises a substantially non-polar solvent.

20. A wipe as claimed in claim 19, wherein the carrier is a volatile silicone oil.

21. A wipe as claimed in claim 20, wherein the carrier is selected from cyclomethicone, octamethylcyclotetrasiloxane, decamethylcyclopentasiloxane, a dimethicone, and mixtures of any of these, or is cyclomethicone.

22. A wipe as claimed in claim 19, 20 or 21, wherein the carrier comprises a surfactant.

23. A wipe as claimed in claim 22, wherein the surfactant is selected from isopropylisostearate, pentaerythratol tetraisostearate, promyristyl propionate, myristyl lactate, oleyl erucate, isopropyl myristate, isocetyl stearate, isopropyl isostearate, mixtures of any of these and other aliphatic esters of fatty acids.

24. A wipe as claimed in any of claims 1-23, wherein the lipophilic emollient is selected from coconut oil, sesame oil, sunflower oil, corn oil, a mineral oil, such as liquid paraffin or a fraction thereof, and mixtures of any of these.

25. A wipe as claimed in claim 24, wherein the lipophilic emollient is coconut oil.

26. A wipe as claimed in any of the preceding claims, impregnated with a pharmaceutically active agent carried in a vehicle which comprises, singularly or in admixture:-
(i) at least one vegetable oil;
(ii) at least one mineral oil;
(iii) at least one volatile silicone oil;
and
(iv) at least one aliphatic ester of a fatty acid.

27. A wipe as claimed in claim 26, wherein the vegetable oil is coconut erachis and/or palm kernal oil, the volatile silicone oil is octamethylcyclotetrasiloxane and/or decamethylcylopentasiloxane and the aliphatic ester of a fatty acid is isopropyl myristate and/or isopropylisostearate.

28. A wipe as claimed in claim 1, wherein the pharmaceutically active agent is 8-methoxypsoralen or trimethoxypsoralen and is included in a concentration of between 0.0125% and 10%.

29. A wipe as claimed in claim 28, wherein the 8-methoxypsoralen or trimethoxypsoralen is included in a concentration of between 0.0125% and 0.1%.

30. A wipe as claimed in claim 1, wherein the pharmaceutically active agent is δ-aminolevulinic acid and is included in a concentration of up to 15%.

31. A wipe as claimed in claim 30, wherein the δ-aminolevulinic acid is in a concentration of up to 10% by weight.

32. A wipe as claimed in any of the preceding claims for use in a topical treatment of a skin condition in combination with electromagnetic radiation, wherein the skin condition is psoriasis, acne vulgaris, alopecia areata, dermatitis herpetiformis, eosinophilic pustular folliculitis, erythrokeratoderma (symmetrical and progressive), chronic lichenoid GVH disease, granuloma annulare, histiocytosis X, ichthyosis linearis circumflexa, lichen planus, pityriasis lichenoides, pityriasis rosea, pityriasis rubra pilaris, pressure sores, pruritis (primary and secondary), seleromyxoedema, subcorneal pustular dermatoses, transient acantholytic dermatoses, or atopic eczema.

33. A wipe as claimed in any of the preceding claims, sealed into an enveloping sachet or pocket.

34. A wipe as claimed in claim 33, wherein the sachet or pocket is formed by sealingly sandwiching a folded and impregnated wipe between two sheets of an aluminium foil/polymer film laminate.

35. A wipe as claimed in claims 1 and 12, for application to the skin of a subject for treating a dermatological disorder, capable of forming a uniform layer of the lipophilic emollient and active agent on the skin of said subject with a thickness of approximately 5µm, said layer being transparent to 80% of a wavelength of therapeutic radiation selected from broad band UV, UVA and UVB, at said thickness, and having a partial pressure less than or equal to 17.5mm Hg at 20°C, said composition being for application prior to said subject receiving a therapeutically effective dose of incident radiation and said layer remaining on said skin, during the administration of the therapeutically effective dose of incident radiation, to direct radiation to the epidermis.

## Patentansprüche

1. Wischtuch, umfassend ein absorptionsfähiges Gewebe oder Faserlies, Tuch oder Gewebesubstrat, imprägniert mit einer erweichenden Zusammensetzung, umfassend ein lipophiles erweichendes und ein pharmazeutisch aktives Mittel, wobei das aktive Mittel eine Substanz ist, die Melanocyten stimuliert, um Melanin zu erzeugen, und/oder bei einer topischen Behandlung einer Hauterkrankung in Kombination mit therapeutischer elektromagnetischer Bestrahlung im Bereich zwischen 220 und 700 nm eingesetzt wird, und ausgewählt wird aus 8-Methoxypsoralen, Trimethylpsoralen, 6-Methoxypsoralen, 3 -Carbethoxypsoralen, Cis-Urocaninsäure, Trans-Urocaninsäure, Gemischen aus Cis- und Trans-Urocaninsäuren und aminooxoaliphatischen Carboxylsäuren, bei denen das aliphatische Gerüst 4-10 Kohlenstoffatome aufweist, die Aminogruppe eine primäre oder eine sekundäre oder eine tertiäre Alkylaminogruppe oder γ oder vorzugsweise δ oder ferner aus dem Carboxykohlenstoff ist, und die Oxogruppe β oder vorzugsweise γ oder ferner aus dem Carboxykohlenstoff ist, die Zusammensetzung eine nichtviskose Flüssigkeit ist, die sich nach dem Aufbringen auf die Haut verteilt und eine im wesentlichen gleichförmige Beschichtung des lipophilen erweichenden und aktiven Mittels bildet, und wobei die Beschichtung keine signifikante Menge an einfallender therapeutischer elektromagnetischer Bestrahlung im Bereich zwischen 220 und 700 nm absorbiert und ausreichend nichtflüchtig ist, um eine ausreichende Zeitlang haften zu bleiben, so daß eine therapeutische wirksame Dosis der genannten elektromagnetischen Strahlung verabreicht werden kann.

2. Wischtuch nach Anspruch 1, bei dem die aminooxoaliphatische Säure eine δ-aminooxoaliphatische Säure ist.

3. Wischtuch nach Anspruch 2, bei dem die aminooxoaliphatische Säure eine δ-Aminolävulinsäure ist.

4. Wischtuch nach Anspruch 2 oder Anspruch 3, bei dem die therapeutische elektromagnetische Strahlung im sichtbaren Spektrum ist.

5. Wischtuch nach Anspruch 4, bei dem die therapeutische Strahlung im Bereich zwischen 600 und 700 nm liegt.

6. Wischtuch nach Anspruch 1, bei dem das pharmazeutische aktive Mittel 8-Methoxypsoralen ist.

7. Wischtuch nach Anspruch 6, bei dem die therapeutische elektromagnetische Strahlung UV im Bereich zwischen 285 und 400 nm ist.

8. Wischtuch nach Anspruch 7, bei dem die therapeutische Strahlung UVA im Bereich zwischen 320 und 400 nm ist.

9. Wischtuch nach Anspruch 1, bei dem die therapeutische elektromagnetische Strahlung Breitband-UV-, UVA- oder UVB-Strahlung, die erweichende Zusammensetzung wasserfrei ist und eine Viskosität zwischen 5 und 2500 Centipoise hat, und das lipophile Erweichungsmittel, wenn es als eine Schicht von 5 µm aufgebracht wird, 10% oder weniger der einfallenden therapeutischen elektromagnetischen Strahlung absorbiert und einen Dampfpartialdruck von 10 mmHg oder weniger bei 20°C hat.

10. Wischtuch nach einem der Ansprüche 1-8, bei dem eine Schicht von 5 µm des lipophilen Erweichungsmittels 20% der eingehenden therapeutischen elektromagnetischen Strahlung oder weniger absorbiert.

11. Wischtuch nach Anspruch 10, bei dem eine Schicht von 5 µm des lipophilen Erweichungsmittels 10% einfallende therapeutische elektromagnetische Strahlung oder weniger absorbiert.

12. Wischtuch nach einem der Ansprüche 1-8, 10 und 11, bei dem die erweichende Zusammensetzung eine Viskosität zwischen 5 und 20.000 Centipoise hat.

13. Wischtuch nach Anspruch 12, bei dem die erweichende Zusammensetzung eine Viskosität zwischen 5 und 2500 Centipoise hat.

14. Wischtuch nach einem der Ansprüche 1-8 und 10-13, bei dem das lipophile Erweichungsmittel einen Dampfpartialdruck von 17,5 mmHg oder weniger bei 20°C hat.

15. Wischtuch nach Anspruch 14, bei dem das lipophile Erweichungsmittel einen Dampfpartialdruck von 10 mmHg oder weniger bei 20°C hat.

16. Wischtuch nach einem der Ansprüche 1-8 und 10-15, bei dem der Dampfpartialdruck einer Beschichtung, die von der erweichenden Zusammensetzung auf der Haut gebildet wurde, 17,5 mmHg oder weniger bei 20°C ist.

17. Wischtuch nach Anspruch 16, bei dem der Dampfpartialdruck einer Beschichtung, die durch die erweichende Zusammensetzung auf der Haut gebildet wurde, 10 mmHg oder weniger bei 20°C ist.

18. Wischtuch nach einem der Ansprüche 1-17, bei dem die erweichende Zusammensetzung ferner einen Träger für das lipophile Erweichungsmittel umfaßt.

19. Wischtuch nach Anspruch 18, bei dem der Träger ein im wesentlichen nichtpolares Lösungsmittel umfaßt.

20. Wischtuch nach Anspruch 19, bei dem der Träger ein flüchtiges Silikonöl ist.

21. Wischtuch nach Anspruch 20, bei dem der Träger ausgewählt wurde aus Cyclomethicon, Octamethylcyclotetrasiloxan, Decamethylcyclopentasiloxan, einem Dimethicon und Gemischen aus diesen, oder Cyclomethicon ist.

22. Wischtuch nach Anspruch 19, 20 oder 21, bei dem der Träger einen grenzflächenaktiven Stoff umfaßt.

23. Wischtuch nach Anspruch 22, bei dem der grenzflächenaktive Stoff ausgewählt wurde aus Isopropylisostearat, Pentaerythratol-Tetraisostearat, Promyristylpropionat, Myristyllactat, Oleylerucat, Isopropylmyristat, Isocetylstearat, Isopropylisostearat, Gemischen aus diesen und anderen aliphatischen Fettsäureestern.

24. Wischtuch nach einem der Ansprüche 1-23, bei dem das lipophile Erweichungsmittel ausgewählt wurde aus Kokosöl, Sesamöl, Sonnenblumenöl, Maisöl, einem Mineralöl wie z.B. flüssigem Paraffin oder einer Fraktion davon, und beliebigen Gemischen aus diesen.

25. Wischtuch nach Anspruch 24, bei dem das lipophile Erweichungsmittel Kokosöl ist.

26. Wischtuch nach einem der vorherigen Ansprüche, imprägniert mit einem pharmazeutisch aktiven Mittel, das in einem Vehikel getragen wird, das allein oder in Mischung folgendes umfaßt:
(i) wenigstens ein Pflanzenöl;
(ii) wenigstens ein Mineralöl;
(iii) wenigstens ein flüchtiges Silikonöl; und
(iv) wenigstens ein aliphatisches Ester einer Fettsäure.

27. Wischtuch nach Anspruch 26, bei dem das Pflanzenöl Kokoserachis und/oder Palmkernöl, das flüchtige Silikonöl Octamethylcyclotetrasiloxan und/oder Decamethylcyclopentasiloxan und das aliphatische Fettsäureester Isopropylmyristat und/oder Isopropylisostearat ist.

28. Wischtuch nach Anspruch 1, bei dem das pharmazeutisch aktive Mittel 8-Methoxypsoralen oder Trimethoxypsoralen ist und in einer Konzentration zwischen 0,0125% und 10% vorliegt.

29. Wischtuch nach Anspruch 28, bei dem das 8-Methoxypsoralen oder Trimethoxypsoralen in einer Konzentration zwischen 0,0125% und 0,1% vorliegt.

30. Wischtuch nach Anspruch 1, bei dem das pharmazeutisch aktive Mittel δ-Aminolävulinsäure ist und in einer Konzentration von bis zu 15% vorliegt.

31. Wischtuch nach Anspruch 30, bei dem die δ-Aminolävulinsäure in einer Konzentration von bis zu 10 Gew.-% vorliegt.

32. Wischtuch nach einem der vorherigen Ansprüche zur Verwendung bei einer topischen Behandlung einer Hauterkrankung in Kombination mit elektromagnetischer Bestrahlung, wobei die Hauterkrankung Psoriase, Akne vulgaris, Alopecia areata, Dermatitis herpetiformis, eosinophile pustulöse Follikulitis, Erythrokeratoderma (symmetrisch oder progressiv), chronische Lichenoid-GVH-Erkrankung, Granuloma anulare, Histiozytose X, Ichthyosis linearis circumflexa, Lichen planus, Pityriasis lichenoides, Pityriasis rosea, Pityriasis rubra pilaris, Druckbrand, Pruritis (primär oder sekundär), Seleromyxoedema, eine subkorneale pustulöse Dermatose, eine transiente acantholytische Dermatose oder Neurodermatitis ist.

33. Wischtuch nach einem der vorherigen Ansprüche, in einem Beutel oder einer Tasche verschlossen.

34. Wischtuch nach Anspruch 33, bei dem der Beutel oder die Tasche hergestellt wird, indem ein gefaltetes und imprägniertes Wischtuch zwischen zwei Aliminiumfolien/Polymerfilm-Lamellen eingeschlossen werden.

35. Wischtuch nach Anspruch 1 und 12 zum Auftragen auf die Haut einer Person zur Behandlung einer dermatologischen Erkrankung, das in der Lage ist, eine gleichförmige Schicht des lipophilen Erweichungsmittels und des aktiven Mittels auf der Haut der genannten Person mit einer Dicke von etwa 5 µm zu bilden, wobei die genannte Schicht zu 80% einer Wellenlänge einer therapeutischen Bestrahlung transparent ist, die ausgewählt wird aus Breitband-UV, UVA und UVB, bei der genannten Dicke, und mit einem Partialdruck von gleich oder kleiner 17,5 mmHg bei 20°C, wobei die genannte Zusammensetzung aufgebracht wird, bevor die genannte Person eine therapeutisch wirksame Dosis einer Eingangsbestrahlung erhält, und wobei die genannte Schicht auf der gennanten Haut während der Verabreichung der therapeutisch wirksamen Dosis der Eingangsbestrahlung bleibt, um die Strahlung auf die Epidermis zu richten.

## Revendications

1. Un tampon textile englobant un textile absorbant tissé ou non tissé, un substrat en étoffe ou en tissu, imprégné d'une composition émolliente comportant un émollient lipophile et un principe pharmaceutique actif, dans lequel le principe actif est une substance efficace pour stimuler les mélanocytes à produire de la mélanine et/ou est efficace pour un traitement local d'un trouble cutané conjointement avec un rayonnement électromagnétique thérapeutique compris dans la plage de 220 à 700 nm, et est sélectionné parmi 8-méthoxypsoralen, triméthylpsoralène, 6-méthoxypsoralen, 3 -carbéthoxypsoralen, acide cis-urocanique, acide trans-urocanique, mélanges d'acides cis- et trans-urocaniques et aminoacides carboxyliques oxoaliphatiques dans lesquels le squelette aliphatique a 4 à 10 atomes de carbone, le groupe amino est primaire, ou est un groupe aminoalkyle secondaire ou tertiaire, et est γ, ou de préférence δ, ou plus éloigné du carboxycarbone et le groupe oxo est β, ou de préférence γ, ou plus éloigné du carboxycarbone, la composition est un liquide non visqueux qui, lorsque appliqué à la peau, s'étale pour produire un revêtement sensiblement uniforme de l'émollient lipophile et du principe actif, et le revêtement n'absorbe pas une quantité significative de rayonnement électromagnétique thérapeutique incident dans la plage de 220 à 700 nm et est suffisamment non volatil pour persister pendant une période suffisamment longue, pour permettre d'administrer une dose thérapeutiquement efficace dudit rayonnement électromagnétique.

2. Un tampon selon la revendication 1, dans lequel l'aminoacide oxoaliphatique est un δ-aminoacide oxoaliphatique.

3. Un tampon selon la revendication 2, dans lequel l'aminoacide oxoaliphatique est un acide δ-aminolévulinique.

4. Un tampon selon la revendication 2 ou 3, dans lequel le rayonnement électromagnétique thérapeutique est dans le spectre visible.

5. Un tampon selon la revendication 4, dans lequel le rayonnement électromagnétique thérapeutique est dans la plage de 600 à 700 nm.

6. Un tampon selon la revendication 1, dans lequel le principe pharmaceutique actif est le 8-méthoxypsoralen.

7. Un tampon selon la revendication 6, dans lequel le rayonnement électromagnétique thérapeutique est UV, dans la plage de 285 à 400 nm.

8. Un tampon selon la revendication 7, dans lequel le rayonnement électromagnétique thérapeutique est UVA, dans la plage de 320 à 400 nm.

9. Un tampon selon la revendication 1, dans lequel le rayonnement électromagnétique thérapeutique est un rayonnement UV, UVA ou UVB à large bande, la composition émolliente est anhydre et a une viscosité entre 5 et 2500 centipoises, et l'émollient lipophile, lorsque étalé en une couche de 5 µm, absorbe 10% ou moins du rayonnement électromagnétique thérapeutique incident et a une pression de vapeur partielle de 10 mmHg ou moins, à 20°C.

10. Un tampon selon l'une quelconque des revendications 1 à 8, dans lequel une couche de 5 µm de l'émollient lipophile absorbe 20% ou moins du rayonnement électromagnétique thérapeutique incident.

11. Un tampon selon la revendication 10, dans lequel une couche de 5 µm de l'émollient lipophile absorbe 10% ou moins du rayonnement électromagnétique thérapeutique incident.

12. Un tampon selon l'une quelconque des revendications 1 à 8, 10 et 11, dans lequel la composition émolliente a une viscosité entre 5 et 20.000 centipoises.

13. Un tampon selon la revendication 12, dans lequel la composition émolliente a une viscosité entre 5 et 2500 centipoises.

14. Un tampon selon l'une quelconque des revendications 1 à 8 et 10 à 13, dans lequel l'émollient lipophile a une pression de vapeur partielle de 17,5 mmHg ou moins à 20°C.

15. Un tampon selon la revendication 14, dans lequel l'émollient lipophile a une pression de vapeur partielle de 10 mmHg ou moins à 20°C.

16. Un tampon selon l'une quelconque des revendications 1 à 8 et 10 à 15, dans lequel la pression de vapeur partielle d'un revêtement, formé par la composition émolliente sur la peau, est 17,5 mmHg ou moins à 20°C.

17. Un tampon selon la revendication 16, dans lequel la pression de vapeur partielle d'un revêtement, formé par la composition émolliente sur la peau, est 10 mmHg ou moins à 20°C.

18. Un tampon selon l'une quelconque des revendications 1 à 17, dans lequel la composition émolliente englobe de plus un véhicule pour l'émollient lipophile.

19. Un tampon selon la revendication 18, dans lequel le véhicule englobe un solvant sensiblement non polaire.

20. Un tampon selon la revendication 19, dans lequel le véhicule est une huile de silicone volatile.

21. Un tampon selon la revendication 20, dans lequel le véhicule est sélectionné parmi cyclométhicone, octaméthylcyclotétrasiloxane, décaméthylcyclopentasiloxane, diméthicone, et des mélanges de ceux-ci, ou est cyclométhicone.

22. Un tampon selon l'une quelconque des revendications 19, 20 ou 21, dans lequel le véhicule englobe un surfactant.

23. Un tampon selon la revendication 22, dans lequel le surfactant est sélectionné parmi l'isopropylisostéarate, le tétraisostéarate de pentaérythritol, le propionate de promyristyle, le lactate de myristyle, l'oléylérucate, le myristate isopropylique, le stéarate isocétylique, l'isostéarate isopropylique, des mélanges de ceux-ci et d'autres esters aliphatiques d'acides gras.

24. Un tampon selon l'une quelconque des revendications 1 à 23, dans lequel l'émollient lipophile est sélectionné parmi l'huile de noix de coco, l'huile de sésame, l'huile de tournesol, l'huile de maïs, une huile minérale telle que la paraffine liquide ou une fraction de celle-ci, et des mélanges de celles-ci.

25. Un tampon selon la revendication 24, dans lequel l'émollient lipophile est l'huile de noix de coco.

26. Un tampon selon l'une quelconque des revendications précédentes, imprégné avec un principe pharmaceutique actif porté dans un véhicule qui englobe un seul ou un mélange des éléments suivants :
(i) au moins une huile végétale ;
(ii) au moins une huile minérale ;
(iii)au moins une huile de silicone volatile ;
et
(iv) au moins un ester aliphatique d'un acide gras.

27. Un tampon selon la revendication 26, dans lequel l'huile végétale est l'huile de noix de coco arachide et/ou une huile de palmiste, l'huile au silicone volatile est l'octaméthylcyclotétrasiloxane et/ou décaméthylcylopentasiloxane et l'ester aliphatique d'un acide gras est le myristate isopropylique et/ou l'isostéarate isopropylique.

28. Un tampon selon la revendication 1, dans lequel le principe pharmaceutique actif est le 8-méthoxypsoralen ou le triméthoxypsoralen et est inclus à une concentration entre 0,0125% et 10%.

29. Un tampon selon la revendication 28, dans lequel le 8-méthoxypsoralen ou le triméthoxypsoralen est inclus en une concentration entre 0,0125% et 0,1%.

30. Un tampon selon la revendication 1, dans lequel le principe pharmaceutique actif est l'acide δ-aminolévulinique et est inclus à une concentration maximale de 15%.

31. Un tampon selon la revendication 30, dans lequel l'acide δ-aminolévulinique est à une concentration maximale de 10% en poids.

32. Un tampons selon l'une quelconque des revendications précédentes, pour l'utilisation dans le traitement local d'un trouble cutané conjointement avec un rayonnement électromagnétique, dans lequel le trouble cutané est le psoriasis, l'acné vulgaire, la pelade, une dermatite herpétiforme, la folliculite pustulaire à éosinophiles, l'érythrokératoderme (symétrique et progressif), la réaction GCH lichéniforme chronique, le granulome annulaire, l'histiocytose X, ichthyosis linearis circumflexa, le lichen plan, le parapsoriasis en gouttes, le pityriasis rosé (de Gibert), le pityriasis rubra pilaire, les escarres de décubitus, les prurits (primaire et secondaire), le scléromyxoedème, les dermatoses pustuleuses sous-cornéennes, les épidermolyses acanthosiques fugaces, ou l'eczéma atopique.

33. Un tampon selon l'une quelconque des revendications précédentes, conditionné dans un sachet ou une pochette d'enveloppement étanche.

34. Un tampon selon la revendication 33, dans lequel le sachet ou la pochette est formé(e) en plaçant en sandwich de manière étanche un tampon plié et imprégné entre deux feuilles d'un stratifié feuille d'aluminium/film de polymère.

35. Un tampon selon les revendications 1 et 12, pour application à la peau d'un sujet pour le traitement d'un trouble dermatologique, capable de former une couche uniforme de l'émollient lipophile et du principe actif sur la peau dudit sujet avec une épaisseur d'environ 5 µm, ladite couche étant transparente à 80% d'une longueur d'onde d'un rayonnement thérapeutique sélectionné parmi les UV, UVA et UVB à large bande, à ladite épaisseur, et ayant une pression partielle inférieure ou égale à 17,5 mmHg à 20°C, ladite composition étant prévue pour l'application avant la réception, par ledit sujet, d'une dose thérapeutiquement efficace de rayonnement incident, et ladite couche demeurant sur ladite peau pendant l'administration de la dose thérapeutiquement efficace du rayonnement incident, pour diriger le rayonnement vers l'épiderme.
